## Europäisches Patentamt
## European Patent Office
## Office européen des brevets

(19)

(11) Numéro de publication : **0 133 079**
**B1**

(12)

# FASCICULE DE BREVET EUROPÉEN

(45) Date de publication du fascicule du brevet :
05.04.89

(21) Numéro de dépôt : 84401408.4

(22) Date de dépôt : 03.07.84

(51) Int. Cl.⁴ : **C 12 P 21/04** // (C12P21/04,
C12R1:465, C07K5:06)

(54) Procédé pour la préparation et l'isolement du nosiheptide.

(30) Priorité : 04.07.83 JP 121273/83

(43) Date de publication de la demande :
13.02.85 Bulletin 85/07

(45) Mention de la délivrance du brevet :
05.04.89 Bulletin 89/14

(84) Etats contractants désignés :
AT BE CH DE FR GB IT LI LU NL SE

(56) Documents cités :
EP-A- 0 073 329
CHEMICAL ABSTRACTS, vol. 96, 1982, page 505,
abrégé no. 33357g, Columbus, Ohio, US
EXPERIENTIA, vol. 36, 1980, pages 414-416, Birkhäuser Verlag, Basel, CH; F. BENAZET et al.: "Nosiheptide, a sulfur-containing peptide antibiotic isolated
from Streptomyces actuosus 40037"

(73) Titulaire : RHONE-POULENC SANTE
20, avenue Raymond Aron
F-92160 Antony (FR)

(72) Inventeur : Morioka, Satoshi MITSUBISHI CHEMICAL
IND. LTD.
Central Research No 1000, Kamoshida-cho
Midori-ku Yokohama-shi Kanagawa-ken (JP)
Inventeur : Shida, Makoto MITSUBISHI CHEMICAL
IND. LTD.
Central Research No 1000, Kamoshida-cho
Midori-ku Yokohama-shi Kanagawa-ken (JP)
Inventeur : Suzuki, Nobuyuki MITSUBISHI CHEMICAL
IND. LTD.
Central Research No 1000, Kamoshida-cho
Midori-ku Yokohama-shi Kanagawa-ken (JP)
Inventeur : Sekihara, Kiyoshi MITSUBISHI CHEMICAL
IND. LTD.
Central Research No 1000, Kamoshida-cho
Midori-ku Yokohama-shi Kanagawa-ken (JP)

(74) Mandataire : Pilard, Jacques et al
RHONE-POULENC RECHERCHES Service Brevets
Pharma 25, Quai Paul Doumer
F-92408 Courbevoie Cedex (FR)

EP 0 133 079 B1

**Description**

La présente invention concerne un procédé pour la séparation et l'isolement du nosiheptide qui est un antibiotique ; plus particulièrement l'invention a pour objet un procédé de séparation et d'isolement du nosiheptide à partir d'un moût de fermentation en utilisant un solvant particulier.

Le nosiheptide, appelé également 9671 RP, est un antibiotique utile comme adjuvant d'alimentation animale et qui est produit dans le brevet japonais publié sous le numéro 880/1965.

Il est connu de préparer le nosiheptide par culture d'une souche productrice de nosiheptide appartenant au genre Streptomyces ; cependant la séparation et l'isolement du nosiheptide du milieu de culture n'a pas, jusqu'à présent, été bien étudiée.

Généralement, la séparation et l'isolement d'un antibiotique à partir d'un milieu de culture sont effectués en mélangeant le milieu de culture avec un solvant et en séparant le mycélium insoluble dans le solvant. Dans ce procédé, la sélection du solvant convenable est très importante. Le solvant doit être tel qu'il assure une bonne séparation entre une portion insoluble dans le solvant ayant le mycélium comme constituant principal et le solvant et qu'il ait une bonne capacité de dissolution de l'antibiotique.

Ce solvant varie selon le mycélium et l'antibiotiqe qui doit être séparé. Par exemple, d'après le brevet japonais publié sous le numéro 29157/73, il est connu d'utiliser l'acétone, le méthanol ou l'éthanol pour séparer et isoler la multhiomycine d'un moût de fermentation obtenu par culture d'une souche productrice de multhiomycine appartenant au genre Streptomyces.

Par ailleurs, la méthode utilisant le chloroforme ou un mélange de solvants ayant le chloroforme comme constituant principal est connue (brevet japonais publié sous le numéro 26718/70) pour séparer et isoler la thiopeptine B d'un milieu de fermentation obtenu par culture d'une souche productrice de thiopeptine B appartenant au genre Streptomyces. Cependant, la capacité de tels solvants connus de dissoudre le nosiheptide est trop faible pour obtenir un effet suffisant.

Devant cette situation, l'objet de la présente invention est de fournir un procédé efficace de séparation et d'isolement du nosiheptide. L'objectif est atteint en utilisant comme solvant un éther cyclique dans un procédé de séparation et d'isolement du nosiheptide qui consiste à mélanger le milieu de fermentation obtenu par culture d'une souche productrice de nosiheptide appartenant au genre Streptomyces avec un solvant, à séparer une partie insoluble ayant le mycélium comme constituant essentiel et à séparer le nosiheptide du solvant. La description détaillée de l'invention est la suivante :

Comme souches productrices de nosiheptide sont connues Streptomyces actuosus (NRRL 2954 ; ATCC 25421), appartenant au genre Streptomyces, et ses mutants.

Le milieu de fermentation contenant le nosiheptide est obtenu en cultivant une telle souche selon le procédé décrit dans le brevet japonais publié sous le numéro 880/1965. Le milieu de fermentation contient entre autres des hydrates de carbone, des sels minéraux, qui proviennent de la composition du milieu de culture, le mycélium et le nosiheptide. Le nosiheptide s'accumule à la surface du mycélium principalement composé d'actinomycètes ayant une structure de surface compliquée.

Le procédé de la présente invention est caractérisé par le mélange du milieu de culture (moût) avec un éther cyclique désigné ci-après comme « premier solvant de traitement ».

Les éthers cycliques comprennent le tétrahydrofuranne, le dioxanne et leurs analogues, le tétrahydrofuranne étant préféré.

Comme milieu de culture convenant pour le traitement par le premier solvant peut être utilisé soit un moût ayant subi un traitement de déshydratation par un moyen mécanique tel que la centrifugation ou la filtration, soit un moût brut n'ayant pas subi de traitement de déshydratation.

Il est difficile d'effectuer le traitement mécanique de déshydratation d'un moût dont la structure de surface est compliquée, alors que l'éther cyclique selon l'invention a un effet solubilisant excellent même si un moût de fermentation ayant une forte teneur en eau est utilisé. Ainsi il est économiquement avantageux d'utiliser un moût brut de fermentation lorsque le traitement par le premier solvant est effectué.

La quantité d'éther cyclique utilisé est différente selon les quantités d'eau et de nosiheptide contenues dans le milieu de culture et elle est généralement choisie entre 0,5 et 5 fois en volumes et de préférence entre 1,5 et 2,5 fois en volumes par rapport au volume du milieu de culture.

Le traitement par le premier solvant est généralement réalisé en utilisant un réservoir agité ayant une trappe dans le fond pour éliminer la matière insoluble. Le temps de traitement est habituellement compris entre 0,5 et 3 heures, de préférence entre 1 et 2 heures, et la température est habituellement comprise entre 5 et 80 °C et de préférence entre la température ambiante et 60 °C.

Le pH du milieu de culture utilisé dans le traitement varie selon les conditions de la culture. Il est préférable d'ajuster le pH entre 3 et 10 et de préférence entre 5 et 7 au moment du traitement. Cet ajustement du pH empêche le nosiheptide de passer dans la couche aqueuse et permet un meilleur isolement lors de la séparation de la couche aqueuse pour éliminer les impuretés qui sera décrite plus loin.

Après le traitement par le premier solvant, le mélange du milieu de culture et du solvant est abandonné pour former deux phases, une phase insoluble contenant le mycélium comme constituant essentiel et une solution claire dans laquelle est dissous le nosiheptide (cette solution claire contient beaucoup d'eau et sera désignée par la suite « solution du premier traitement »). Les deux phases sont séparées en éliminant les matières insolubles précipitées par l'orifice au fond du réservoir agité.

Un seul traitement décrit ci-dessus est habituellement suffisant puisque l'éther cyclique utilisé dans la présente invention a une grande capacité pour dissoudre le nosiheptide ; cependant, les matières insolubles séparées peuvent être à nouveau traitées par l'éther cyclique si nécessaire.

Le nosiheptide, dissous dans la solution du premier traitement, est séparé de sa solution. Avant cette séparation, il est préférable d'ajouter et de mélanger un sel minéral ou de préférence un deuxième solvant miscible dans le solvant du premier traitement, mais légèrement soluble ou insoluble dans l'eau de façon à séparer une couche aqueuse de la solution de premier traitement puis à éliminer cette couche aqueuse.

Les sels minéraux sont choisis parmi le chlorure de magnésium, le chlorure de sodium ou le chlorure de calcium. Le benzène, le toluène, le xylène, l'hexane, l'heptane ou le cyclohexane sont utilisés comme solvants non miscibles dans l'eau. La quantité de second solvant utilisée varie selon la quantité d'eau contenue dans la solution de premier traitement ; par exemple la quantité de solvant légèrement miscible dans l'eau représente 0,05 à 0,5 fois en volumes, de préférence 0,25 à 0,35 fois en volumes, le volume de la solution de premier traitement. Lorsque la quantité de solvant est inférieure aux quantités ci-dessus, la quantité totale d'eau contenue dans la solution de premier traitement ne peut pas être séparée et lorsque la quantité de solvant est supérieure, le nosiheptide précipite de sa solution.

Pour que la formation de la couche aqueuse puisse être réalisée dans le réservoir agité, après séparation des matériaux insolubles, le sel minéral ou le solvant non miscible dans l'eau est ajouté et mélangé à la solution de premier traitement dans le réservoir agité à la même température que celle de la solution de premier traitement pendant 1 à 10 minutes.

Les impuretés telles que les hydrates de carbone et les sels minéraux dissous dans le solvant du premier traitement passent en solution dans l'eau et le nosiheptide hautement purifié est séparé directement de la solution du premier traitement.

A ce moment, une faible quantité de nosiheptide est perdue par transfert dans la couche aqueuse ; cependant ceci peut être efficacement évité en ajustant le pH du milieu de culture utilisé dans le traitement par le premier solvant aux valeurs indiquées ci-dessus.

La méthode pour séparer le nosiheptide de la solution de premier traitement peut consister soit à distiller le solvant puis à séparer le nosiheptide mécaniquement, soit à précipiter le nosiheptide en ajoutant un composé tiers au solvant de premier traitement après séparation de la couche aqueuse ; il est préférable d'utiliser comme composé tiers le même solvant que celui qui est utilisé pour la séparation de la couche aqueuse.

La quantité de solvant (composé tiers) utilisée pour séparer le nosiheptide représente entre 0,2 et 1 fois en volume, de préférence entre 0,4 et 0,6 fois en volumes, le volume de la solution de premier traitement après élimination de l'eau et, lorsque l'eau n'est pas séparée, elle représente entre 0,25 et 1,5 fois en volumes, de préférence entre 0,65 et 0,95 fois en volumes, le volume de la solution de premier traitement.

Le traitement est effectué dans les mêmes conditions que celles utilisées pour la séparation de l'eau, c'est-à-dire en ajoutant le solvant dans le réservoir agité après séparation de l'eau.

L'isolement du nosiheptide précipité dans la solution de premier traitement peut être facilement réalisé au moyen de la centrifugation ou de la filtration.

Le nosiheptide ainsi obtenu peut parfois contenir de faibles quantités de métaux lourds tels que le fer, le cobalt, le nickel, le cuivre ou le manganèse. Il est nécessaire, pour éliminer les métaux lourds, de dissoudre le nosiheptide dans un solvant convenable et de traiter la solution par du charbon désactivé, du gel de silice ou une résine échangeuse d'ions.

La séparation des métaux lourds peut aussi être effectuée à partir de la solution de premier traitement après élimination de l'eau.

La présente invention permet de réaliser la séparation et l'isolement du nosiheptide d'un milieu de culture et d'une façon économique en utilisant un solvant spécifique et elle contribue de manière importante au domaine de la production du nosiheptide.

Le procédé selon la présente invention est illustrée par l'exemple suivant donné à titre non limitation.

## Exemple

Un réservoir agité avec une trappe dans le fond est chargé avec 100 parties (en volumes) d'un milieu de culture (moût) obtenu par culture de Streptomyces actuosus, et avec 200 parties (en volumes) de tétrahydrofuranne. Le mélange est effectué par agitation à 60 °C pendant 1 heure.

La composition du milieu de culture est la suivante :

| | |
|---|---|
| mycélium | 7,5 % (en poids) |
| hydrate de carbone | 2 % |
| sels minéraux | 0,5 % |
| nosiheptide | 0,5 % |
| eau | 89,5 % |

Le pH est de 5,6.

On laisse reposer pendant 5 minutes pour précipiter les matières insolubles. Les matières insolubles sont éliminées par la trappe. La quantité de matières insolubles est de 75 parties (en volumes) et celle de la solution du premier traitement (solution dans le tétrahydrofuranne) dans le réservoir est de 225 parties (en volumes).

L'analyse par chromatographie liquide montre que la solution contient 2,11 % en poids de nosiheptide.

On ajoute 60 parties (en volumes) d'heptane dans le réservoir, agite pendant 5 minutes, abandonne pendant 10 minutes puis élimine la couche aqueuse inférieure formée par la trappe [la quantité d'eau éliminée est de 60 parties (en volumes), et le solvant de premier traitement qui est conservé représente 225 parties (en volumes)]. Les taux d'élimination des hydrates de carbone et des sels minéraux sont respectivement de 95 % et de 99 % en poids.

L'analyse de chaque constituant, pour déterminer le taux d'élimination, est effectué de la façon suivante :

carbohydrates :

Après addition d'eau, la solution de premier traitement est filtrée pour séparer le nosiheptide précipité et le résidu obtenu après distillation et séchage du filtrat est dissous dans l'eau. Après addition de réactifs colorés, on mesure l'absorption.

sels minéraux :

L'analyse par absorption atomique est effectuée en utilisant la solution de premier traitement.

On ajoute à nouveau 100 parties (en volumes) d'heptane à la solution de tétrahydrofuranne après élimination de l'eau, puis on agite pendant 10 minutes. Le contenu du réservoir est alors soumis à une séparation par centrifugation et le nosiheptide est séparé.

La concentration du nosiheptide dans le solvant est à l'état de traces et la pureté du nosiheptide isolé est de 95 % en poids d'après l'analyse par chromatographie liquide.

## Exemple de référence

La solubilité du nosiheptide, issu du milieu de culture utilisé dans l'exemple 1, dans différents solvants est déterminée et les résultats sont rassemblés dans le tableau 1.

Le taux de solubilité est déterminé en accord avec les normes japonaises prescrites dans « Ministerial ordinance concerning the specification on the component of feed and feed additive ».

Tableau 1

| Solvant | Solubilité |
|---|---|
| éthanol | très légèrement soluble |
| isopropanol | légèrement soluble |
| alcool anmylique | légèrement soluble |
| toluène | légèrement soluble |
| chloroforme | légèrement soluble |
| dichlorométhane | très légèrement soluble |
| dichloroéthane | très légèrement soluble |
| acétate d'éthyle | très légèrement soluble |
| acétate d'isobutyle | très légèrement soluble |
| acétone | légèrement soluble |
| méthylisobutylcétone | légèrement soluble |
| tétrahydrofuranne | soluble |

## Revendications

1. Procédé pour la séparation et l'isolement du nosiheptide par mélange d'un solvant avec le milieu de culture obtenu par culture d'une souche productrice de nosiheptide appartenant au genre Streptomyces, puis par séparation d'une partie insoluble contenant essentiellement le mycélium, et enfin par isolement du nosiheptide, caractérisé en ce que le solvant est un éther cyclique.

2. Procédé pour la séparation et l'isolement du nosiheptide selon la revendication 1 caractérisé en ce que l'éther cyclique est le tétrahydrofuranne.

3. Procédé pour la séparation et l'isolement du nosiheptide selon l'une des revendications 1 ou 2 caractérisé en ce que le volume de solvant est de 0,5 à 5 fois celui du milieu de culture.

4. Procédé pour la séparation et l'isolement du nosiheptide selon l'une des revendications 1, 2 ou 3 caractérisé en ce que, au milieu de culture traité par le premier solvant, on ajoute un second solvant, totalement miscible dans le premier solvant et légèrement miscible dans l'eau, en quantité représentant entre 0,05 et 0,5 fois le volume du premier solvant, puis sépare la couche aqueuse et isole le nosiheptide de sa solution après élimination des matières insolubles.

## Claims

1. Process for the separation and isolation of nosiheptide, by mixing a solvent with the culture medium obtained by culturing a strain that produces nosiheptide belonging to the genus Streptomyces, by then separating an insoluble portion essentially containing the mycelium, and finally by isolating the nosiheptide, characterized in that the solvent is a cyclic ether.

2. Process for the separation and isolation of nosiheptide according to Claim 1, characterized in that the cyclic ether is tetrahydrofuran.

3. Process for the separation and isolation of nosiheptide according to one of Claims 1 and 2, characterized in that the volume of solvent is from 0.5 to 5 times that of the culture medium.

4. Process for the separation and isolation of nosiheptide according to one of Claims 1, 2 and 3, characterized in that, to the culture medium treated with the first solvent, a second solvent, which is completely miscible with the first solvent and slightly miscible with water, is added in an amount representing between 0.05 and 0.5 times the volume of the first solvent, the aqueous layer is then separated off and the nosiheptide is isolated from its solution after removal of insoluble matter.

## Patentansprüche

1. Verfahren zum Abtrennen und Isolieren von Nosiheptid durch vermischen eines Lösungsmittels mit dem Kulturmilieu, das durch Kultur eines Nosiheptid erzeugenden Stammes der Gattung Streptomyces erhalten wurde, anschließende Abtrennung eines hauptsächlich das Mycel enthaltenden unlöslichen Teiles und abschließende Isolierung des Nosiheptids, dadurch gekennzeichnet, daß das Lösungsmittel ein cyclischer Äther ist.

2. Verfahren zum Abtrennen und Isolieren von Nosiheptid nach Anspruch 1, durch gekennzeichnet, daß der cyclische Äther Tetrahydrofuran ist.

3. Verfahren zum Abtrennen und Isolieren von Nosiheptid nach einem der Ansprüche 1 oder 2, dadurch gekennzeichnet, daß das Volumen des Lösungsmittels das 0,5- bis 5-fache Volumen des Kulturmediums ist.

4. Verfahren zum Abtrennen und Isolieren von Nosiheptid nach einem der Ansprüche 1, 2 oder 3, dadurch gekennzeichnet, daß man dem mit dem ersten Lösungsmittel behandelten Kulturmilieu ein mit dem ersten Lösungsmittel vollständig und mit Wasser schwach mischbares zweites Lösungsmittel in einer Menge zusetzt, die dás 0,05- bis 0,5-fache Volumen des ersten Lösungsmittels ausmacht, dann die wässerige Schicht abtrennt und das Nosiheptid von seiner Lösung nach Entfernung der unlöslichen Materialien abtrennt.